# EUROPEAN PATENT APPLICATION

(11) **EP 1 172 113 A1**
(43) Date of publication of application: **16.01.2002**
(21) Application number: 00915408.9
(22) Date of filing: 07.04.2000
(51) Int. Cl.: A61K 38/18, A61K 45/00, A61P 25/02, A61P 9/00, A61P 1/00, A61P 13/00, A61P 3/10, A61P 3/08, A61P 11/00, A61P 5/00, A61P 27/02

(54) **REMEDIES FOR AUTONOMIC NEUROPATHY**

(30) Priority: 16.04.1999 JP 10960399; 16.04.1999 JP 10960499; 15.06.1999 JP 16830599
(71) Applicant: Sumitomo Pharmaceuticals Company, Limited, Osaka-shi, Osaka 541-8510 (JP)
(72) Inventor: NONOMURA, Takeshi, Nishinomiya-shi, Hyogo 662-0831 (JP); TSUCHIDA, Atsushi, Nara-shi, Nara 631-0035 (JP); TAIJI, Mutsuo, Takatsuki-shi, Osaka 569-1044 (JP)
(74) Representative: Cresswell, Thomas Anthony
(86) International application number: JP0002265
(87) International publication number: WO0062797

(57) **Abstract**

An agent for treatment of autonomic neuropathy, an agent for treating or inhibiting autonomic imbalance, an agent for improving energy metabolism or an agent for improving thermogenesis containing as the active ingredient a neurotrophic factor (BDNF, etc.), or a trk receptor agonist, whereby autonomic neuropathy induced by various causes, in particular, orthostatic hypotension, thermoregulation disorder, autonomic imbalance, energy metabolic abnormality and body temperature abnormality caused by sympathetic nerve hypofunction can be efficaciously prevented and treated.

## Description

### TECHNICAL FIELD

The present invention relates to an agent for treatment of autonomic neuropathy or autonomic imbalance. More particularly, the present invention relates to an agent for improving energy metabolism or an agent for improving thermogenesis.

### BACKGROUND ART

The autonomic nerve is a nervous system for maintaining the homeostasis of living body by controlling internal organs, blood vessels, secretory glands, etc., which are essential to homeostasis, and it distributes almost all of the organs of entire body. The autonomic nerve consists of the sympathetic nervous system and the parasympathetic nervous system, and these nervous systems act competitively on each other and control each organ, but once the balance between these nervous systems is lost, autonomic neuropathy is induced, and various pathologies appear.

Autonomic neuropathy may be induced by various causes, and its representative underlying disease is exemplified such as (1) central diseases: essential orthostatic hypotension, Shy-Drager Syndrome (i.e., autonomic neurologic symptom having mainly orthostatic hypotension, urinary disturbance), Parkinson's disease, epilepsy, disseminated sclerosis, cerebrovascular disease, (2) peripheral diseases: diabetes mellitus, familial amyloidosis, Guillain-Barre syndrome, Fabry's disease, addictive (drug, alcohol) neuropathy, thyroid abnormalities, (3) damaged spinal cord, etc. In addition, there are other autonomic neuropathies such as ones induced by aging or ones with no specific cause, for example, autonomic imbalance (see Yukihisa Shibayama, Current Therapy, vol. 16, no. 4, p. 89-90 (1998)).

Autonomic neuropathy is, for example, circulatory diseases (e.g., orthostatic hypotension (blackout), decreased ability to exercise, cardiac infarction sine dolore, angina pectoris sine dolore, etc.), gastrointestinal disorders (e.g., esophageal mobility abnormality, decreased gastric mobility (gastroparalysis), convulsion of pyloric portion of stomach, intestinal motility abnormality (diarrhea), decreased intestinal motility (constipation), decreased gallbladder contraction (cholecystitis), colon anus dysfunction (incontinence of feces), gluten sensitive enteropathy, etc.), urinary disorders (e.g., bladder diseases (atonic bladder, incontinence after urination, etc.), impotence, abnormal ejaculation, etc.), respiratory disorders (e.g., respiratory dysfunction, sleep apnea syndrome, etc.), thermoregulation disorders (e.g., sweat gland abnormality (perspiration abnormality), vasomotor abnormality (e.g., vasoconstriction, vasodilation disorder, nervous edema, etc.)), pupillary disorders (e.g., contracted pupil, mydriatic disorder, Argyll-Robertson syndrome, etc.), neuroendocrine disorders (e.g., pancreatic PP (polypeptide) hormonal secretion abnormality, somatostatin secretion abnormality, motilin, GIP secretion abnormality, gastrin secretion abnormality, norepinephrine secretion abnormality (uprise disorder), parathyroid hormone secretion abnormality, decreased atrial natriuretic peptide (ANP), asymptomatic hypoglycemia, etc.), etc.

In addition to the treatment of underlying diseases, symptomatic therapy has been applied to each autonomic symptom in the treatment of autonomic neuropathy. Namely, sympathetic agents, prostaglandin synthetase inhibitors, mineralocorticoids, etc. are employed to the treatment of orthostatic hypotension, and cholinergic drugs or dopamine antagonists are employed to the improvement of gastric mobility function. Further, in the treatment of urinary disturbance, detrusor muscle relaxants are employed ([Today's Medical Guideline, we are practicing in this way, 1998 version], published by Igaku Shoin, under the editorship of Shigeaki Hinohara and Masakazu Abe, p. 242).

However, there has not been known a medicament being able to generally treating autonomic neuropathy, which is induced by various causes. For example, a conventional agent for treatment of orthostatic hypotension as mentioned above (e.g., sympathetic agents, etc.) may possibly decrease gastric mobility function, and on the contrary, an agent for improving gastric mobility function (e.g., dopamine antagonist, cholinergic drug, etc.) may lower disillusion degree, and may have a tendency of aggravating hypotension or hypothermia. In addition, with respect to the thermoregulation abnormality, which is induced by many factors such as metabolism of carbohydrate or lipid at the peripheral organs or perspiration action, any specifically effective method for treatment thereof other than the treatment of underlying diseases has not been known ([Endocrine metabolic disorder and body temperature]; Rinsho Kango (i.e., clinical nursing), vol. 8, No. 14, p. 2202-2206 (1982)).

The living body maintains the constant composition of substances, but in fact, it is in dynamic equilibrium conditions, wherein substances are partially but constantly exchanged to the external world. The chemical change occurring in the living body is called substance metabolism. Since the incomings and outgoings of substances mean the incomings and outgoings of energy, this same fact can be said as energy metabolism from the viewpoint of energy.

The living body can exist by taking nutrients being consisting of high molecules and being in abundant of chemical energy, followed by oxidizing them or converting them to mechanical energy for working, or converting them to heat energy and keeping body temperature, during which there is a balance between the energy intake and the energy consumption, according to the first law of thermodynamics (law of conservation of energy). Keeping this balance is considered as healthy condition.

If this balance is lost, for example, if there has been a disparity between the energy intake and the energy consumption for a long period, then the body fat is increased or decreased, and as a result, obesity or emaciation may be induced. However, if this imbalance continues only for a rather short period, even though the body weight is temporarily increased or decreased by this imbalance, the body weight can recover to the original body weight in the long view. Further, even though the energy intake is controlled by increasing or decreasing the amount of food to be eaten, the increase or decrease in energy intake cannot be the increase or decrease in energy consumption, and hence, the imbalance, i.e., the energy metabolic abnormality, may be considered as a cause for obesity or emaciation.

Further, the energy metabolic abnormality may induce not only obesity but also various diseases represented by life-style related diseases. Life-style related diseases have been considered to be caused by various metabolic abnormalities induced by a complicated and multiple arrangement of dietary habit, exercise habit, or inherited factors.

Therefore, it is indicated that a drug normalizing the amount of the energy usage, i.e., the energy metabolism, can possibly make not only a medicament for improving obesity but also a medicament for prevention or normalization of diseases induced by obesity or metabolic disorder to healthy conditions.

Diseases induced by obesity or metabolic disorder include, for example, diabetes mellitus (e.g., type 2 diabetes mellitus, type 1 diabetes mellitus), hyperlipemia, hypo-HDL-cholesterolemia, and arteriosclerosis, hypertension, hypothermia, etc.

Especially, diabetes mellitus can be often found in persons to be fat or persons who were fat in the past. Besides, it is true that there is a high tendency of type 2 diabetes mellitus among persons to be fat. It is not sure at the moment that there is type 2 diabetes mellitus which is induced and onset by obesity as a direct cause therefor, but it is beyond question that obesity is one of the causes for type 2 diabetes mellitus, and further, it is also apparent that metabolic hypofunction is one of the causes for diabetes mellitus even though obesity is not accompanied.

In addition, when comparing the fatality rates between causes of death of persons with obesity, diabetes mellitus is ranked first, and its fatality rate is about 4 times higher than that of persons with normal body weight. Recently, it has been a focus of attention that insulin resistant conditions accompanying type 2 diabetes mellitus may have a high risk of inducing other diseases (insulin resistant syndrome) such as hypertension, hyperlipemia, arteriosclerosis, etc.

Hyperlipemia is a disease wherein the level of serum lipids, mainly total cholesterol or neutral fat (triglyceride) in the blood, is higher than the normal level. When this condition lasts for a long period, various symptoms appear, and among them, arteriosclerosis is the most serious problem. Recently, insulin resistant syndrome has been a focus of attention, which is a disease of combining various pathologies such as hyperlipemia, hypertension and diabetic mellitus. If arteriosclerosis greatly infects on the prognosis of obesity, then it will be necessary to actively approach and study hyperlipemia, which is easily accompanied by obesity.

Further, with respect to lipid metabolic abnormality accompanying obesity, not only hypertriglycemia but also hypo-HDL-cholesterolemia is also important from a viewpoint of the prevention of arteriosclerosis. In addition, if lipid metabolic function is insufficient enough, there is a high tendency of hyperlipemia even though obesity is not accompanied.

It has been proved by various studies that obesity and hypertension closely relate each other. It is usual that a person with obesity may suffer from hypertension, and that a patient with hypertension may often suffer from obesity. From epidemiological surveys, it is said that hypertension of persons with obesity is about 2 to 3 times higher than that of persons without obesity. Further, it is apparent that the blood pressure of a person with obesity and being suffering from hypertension can be decreased by the weight loss, regardless of the sodium chloride intake.

Recently, insulin resistance, which is one of metabolic disorders, has been a focus of attention as a contributing factor of hypertension, together with impaired glucose tolerance and hyperlipemia (insulin resistant syndrome). Therefore, in the treatment of hypertension, it is important to not only reduce the blood pressure but also to ameliorate underlying diseases thereof such as obesity, metabolic disorders and insulin resistance.

The prevention and treatment of diabetes mellitus and other diseases relating insulin resistance (impaired glucose tolerance and hyperlipemia) have been carried out by a combination of dietary therapy and exercise therapy, but it is not satisfactory yet.

As explained above, it has been desired to develop an agent for ameliorating obesity, or an agent for prevention or treatment of diseases induced by obesity or metabolic disorders such as diabetes mellitus (e.g., type 2 diabetes mellitus, type 1 diabetes mellitus), hyperlipemia, hypo-HDL-cholesterolemia, arteriosclerosis, hypertension, etc.

On the other hand, it has been known that metabolic function does not sufficiently act during illness, after illness or operation, or physical fatigue to induce hypoalimentation. Under these conditions, it has been desired to develop an agent for promoting recovery by activating metabolism and promoting energy production or an agent for improving physical conditions.

Further, in diseases wherein the thermogenesis is not sufficient because of metabolic dysfunction, it has been desired to develop an agent for preventing decreased body temperature, for protecting the tissues, and for improving the blood circulation, etc.

On the other hand, neurotrophic factor is a generic name for proteins, which are provided from target cells or neurons and glia cells and Schwann cells in the living body. They show activities to maintain the survival and differentiation of neurons, and are classified into many types according to the kinds of nerves or receptors to function. Among them, proteins being known as neurotrophins have high structural homology with each other and form a family. The typical examples thereof are neurotrophins such as nerve growth factor (hereinafter, abbreviated as NGF), brain-derived neurotrophic factor (hereinafter, abbreviated as BDNF), neurotrophin 3 (hereinafter, abbreviated as NT-3), neurotrophin 4 (hereinafter, abbreviated as NT-4), neurotrophin 5 (hereinafter, abbreviated as NT-5), or neurotrophin 6 (NT-6); ciliary neurotrophic factor (hereinafter, abbreviated as CNTF); glia cell-derived neurotrophic factor (hereinafter, abbreviated as GDNF), etc. In addition, neurotrophins are known to act as a specific ligand of receptors (trkA, trkB and/or trkC), which are the products of p-75 and trk genes (cf. Takeshi NONOMURA, Hiroshi HATANAKA; Jikken Igaku, vol. 13, p. 376 (1995)).

Neurotrophic factors have been studied with respect to their medical use as a therapeutic agent for treating a patient of neurodegenerative diseases. For example, Society for Neuroscience, vol. 21, p. 1535 (1995), A.P. Mizisin et al. discloses the pharmacological activity of BDNF on diabetic peripheral neuropathy, but this literature merely suggests the possible pharmacological activity of BDNF on diabetic peripheral neuropathy based on the finding that BDNF inhibits the reduction of motor nerve conduction. WO 93-1300 (Regeneron Pharmaceuticals Inc.) discloses various applications of neurotrophic factors onto various motor nerve degenerative diseases. In addition, W.E. Snider, Nature Neuroscience, vol. 1, no. 1, p. 5-6 (1998.5) discloses the involvement of BDNF in skin tactile sense.

### DISCLOSURE OF INVENTION

As mentioned above, an agent for improving autonomic neuropathy or autonomic imbalance or an agent for improving energy metabolism or thermogenesis has been desired in the medical field.

When the present inventors administered BDNF, one of neurotrophic factors, to one of animal models for autonomic neuropathy, i.e., animal models for type 2 diabetes mellitus (obesity and hyperglycemia), the following new knowledge was obtained.

The present inventors have found that neurotrophic factors can (1) increase the norepinephrine content in the peripheral organs, which is reduced in animal models for type 2 diabetes mellitus, (2) ameliorate the decreased functions of the peripheral organs, and (3) raise the decreased body temperature and keep it normal, and have accomplished the present invention.

The present inventors have further intensively studied, and have found that neurotrophic factors can improve the energy metabolism, and have accomplished the present invention. More concretely, the present inventors have found that neurotrophic factors accelerate the thermogenesis and the oxygen consumption, by which can show an activity of improving the energy metabolism, and they have accomplished to prepare an agent for improving energy metabolism, and an agent for prevention diseases induced by the energy metabolic disorders, and an agent for improving thermogenesis, comprising as the active ingredient a neurotrophic factor.

Namely, the present invention relates to agents for treatment of autonomic neuropathy or autonomic imbalance, which comprises as the active ingredient a neurotrophic factor. More particularly, the agents for treatment of autonomic neuropathy of the present invention, which comprises as the active ingredient a neurotrophic factor, are effective on circulatory diseases (e.g., orthostatic hypotension (blackout), decreased ability to exercise, cardiac infarction sine dolore, angina pectoris sine dolore, etc.), gastrointestinal disorders (e.g., esophageal mobility abnormality, decreased gastric mobility (gastroparalysis), convulsion of pyloric portion of stomach, intestinal motility abnormality (diarrhea), decreased intestinal motility (constipation), decreased gallbladder contraction (cholecystitis), colon anus dysfunction (incontinence of feces), gluten sensitive enteropathy, etc.), urinary disorders (e.g., bladder diseases (atonic bladder, incontinence after urination, etc.), impotence, abnormal ejaculation, etc.), respiratory disorders (e.g., respiratory dysfunction, sleep apnea syndrome, etc.), thermoregulation disorders (e.g., sweat gland abnormality (perspiration abnormality), vasomotor abnormality (e.g., vasoconstriction, vasodilation disorder, nervous edema, etc.)), pupillary disorders (e.g., contracted pupil, mydriatic disorder, Argyll-Robertson syndrome, etc.), neuroendocrine disorders (e.g., pancreatic PP (polypeptide) hormonal secretion abnormality, somatostatin secretion abnormality, motilin, GIP secretion abnormality, gastrin secretion abnormality, norepinephrine secretion abnormality (uprise disorder), parathyroid hormone secretion abnormality, decreased atrial natriuretic peptide (ANP), asymptomatic hypoglycemia, etc.), etc.

From the fact that neurotrophic factors ameliorate the decreased conditions of normal energy metabolism, the agent for improving energy metabolism of the present invention can be used in the treatment of lipid metabolic disorders (e.g., hyperlipidemia, hyperlipoproteinemia (primary hyperlipoproteinemia (type I hyperlipoproteinemia, type II hyperlipoproteinemia, type IIb hyperlipoproteinemia, type VI hyperlipoproteinemia, type V hyperlipoproteinemia, etc.) etc.) etc.), or derangement of carbohydrate metabolism. Besides, the agent for improving thermogenesis of the present invention can also be used as an agent for improving cold constitution or hypothermia, an agent for inhibiting decreased body temperature in cold place, or as an agent for normalizing decreased body temperature, or as an agent for prevention of frostbite. Further, the agent for improving energy metabolism of the present invention can also be used as an agent for improving physical constitution, which can increase the energy production efficiency in malnutrition conditions during illness, after illness or operation, or as a revitalizer during decreased physical strength. Further, the agent for improving energy metabolism of the present invention can also be used, for example, as an agent for improving or activating the energy metabolism of skeletal muscles, or as an agent for improving or activating the energy metabolism of brown adipose tissue. More further, the agent for improving energy metabolism or the agent for improving thermogenesis of the present invention, which comprises as the active ingredient a neurotrophic factor, is effective on diabetic patients having decreased insulin deficiency, insulin resistance or insulin hyposensitivity.

More particularly, the present invention relates to the following:
1. An agent for treatment of autonomic neuropathy, which comprises as the active ingredient a neurotrophic factor;
2. An agent for treatment of autonomic imbalance, which comprises as the active ingredient a neurotrophic factor;
3. The agent for treatment of autonomic imbalance according to above 2, wherein the autonomic imbalance is induced by autonomic neuropathy;
4. The agent for treatment of autonomic neuropathy or autonomic imbalance according to the above 1 or 3, wherein the autonomic neuropathy is a circulatory disease, respiratory disorder, gastrointestinal disorder, urinary disorder, thermoregulation disorder, pupillary disorder, or neuroendocrine disorder;
5. The agent for treatment of autonomic neuropathy or autonomic imbalance according to the above 4, wherein the circulatory disease is orthostatic hypotension (blackout), decreased ability to exercise, cardiac infarction sine dolore, or angina pectoris sine dolore;
6. The agent for treatment of autonomic neuropathy or autonomic imbalance according to the above 4, wherein the gastrointestinal disorder is esophageal mobility abnormality, decreased gastric mobility (gastroparalysis), convulsion of pyloric portion of stomach, intestinal motility abnormality (diarrhea), decreased intestinal motility (constipation), decreased gallbladder contraction (cholecystitis), colon anus dysfunction (incontinence of feces), or gluten sensitive enteropathy;
7. The agent for treatment of autonomic neuropathy or autonomic imbalance according to the above 4, wherein the urinary disorder is a urinary bladder disease (atonic bladder, incontinence after urination), impotence, or abnormal ejaculation;
8. The agent for treatment of autonomic neuropathy or autonomic imbalance according to the above 4, wherein the respiratory disorder is a respiratory dysfunction or sleep apnea syndrome;
9. The agent for treatment of autonomic neuropathy or autonomic imbalance according to the above 4, wherein the thermoregulation disorder is sweat gland abnormality (perspiration abnormality) or vasomotor abnormality (vasoconstriction, vasodilation disorder, nervous edema);
10. The agent for treatment of autonomic neuropathy or autonomic imbalance according to the above 4, wherein the pupillary disorder is contracted pupil, mydriatic disorder, or Argyll-Robertson syndrome;
11. The agent for treatment of autonomic neuropathy or autonomic imbalance according to the above 4, wherein the neuroendocrine disorder is pancreatic PP (polypeptide) hormonal secretion abnormality, somatostatin secretion abnormality, motilin, GIP secretion abnormality, gastrin secretion abnormality, norepinephrine secretion abnormality (uprise disorder), parathyroid hormone secretion abnormality, decreased atrial natriuretic peptide (ANP), or asymptomatic hypoglycemia;
12. An agent for treatment of autonomic neuropathy or autonomic imbalance induced by stress, which comprises as the active ingredient a neurotrophic factor;
13. An agent for treatment of autonomic neuropathy or autonomic imbalance induced by diabetes mellitus, which comprises as the active ingredient a neurotrophic factor;
14. An agent for treatment of autonomic neuropathy or autonomic imbalance induced by menopausal syndrome, which comprises as the active ingredient a neurotrophic factor;
15. The agent for treatment of autonomic neuropathy according to the above 1, wherein the autonomic neuropathy is diabetic autonomic neuropathy;
16. The agent for treatment of autonomic neuropathy according to the above 1, wherein the autonomic neuropathy is orthostatic hypotension;
17. An agent for inhibiting norepinephrine content decrease in peripheral tissue, which comprises as the active ingredient a neurotrophic factor;
18. An agent for inhibiting norepinephrine content decrease in peripheral tissue according to the above 17, wherein the peripheral tissue is brown adipose tissue, heart and/or muscles;
19. An agent for improving decreased peripheral organ function, which comprises as the active ingredient a neurotrophic factor;
20. The agent according to any one of the above 1 to 19, wherein the neurotrophic factor of the active ingredient is NGF (nerve growth factor), BDNF (brain-derived neurotrophic factor), NT-3 (neurotrophin 3), NT-4 (neurotrophin 4), NT-5 (neurotrophin 5), NT-6 (neurotrophin 6), CNTF (ciliary neurotrophic factor), or GDNF (glia cell-derived neurotrophic factor);
21. The agent according to any one of the above 1 to 19, wherein the neurotrophic factor of the active ingredient is BDNF (brain-derived neurotrophic factor);
22. The agent according to any one of the above 1 to 19, wherein the neurotrophic factor of the active ingredient is CNTF (ciliary neurotrophic factor);
23. The agent according to any one of the above 1 to 19, wherein the neurotrophic factor of the active ingredient is NT-3 (neurotrophin 3);
24. The agent according to any one of the above 1 to 19, wherein the neurotrophic factor of the active ingredient is NT-4 (neurotrophin 4);
25. The agent according to any one of the above 1 to 19, wherein the neurotrophic factor of the active ingredient is NT-5 (neurotrophin 5);
26. The agent according to any one of the above 1 to 19, wherein the neurotrophic factor of the active ingredient is NT-6 (neurotrophin 6);
27. The agent according to any one of the above 1 to 19, wherein the neurotrophic factor of the active ingredient is NGF (nerve growth factor);
28. The agent according to any one of the above 1 to 19, wherein the neurotrophic factor of the active ingredient is GDNF (glia cell-derived neurotrophic factor);
29. The agent according to any one of the above 1 to 19, wherein the neurotrophic factor of the active ingredient is a trkA, a trkB and/or a trkC receptor agonist;
30. An agent for improving energy metabolism, which comprises as the active ingredient a neurotrophic factor;
31. The agent for improving energy metabolism according to the above 30, wherein the neurotrophic factor is NGF (nerve growth factor), BDNF (brain-derived neurotrophic factor), NT-3 (neurotrophin 3), NT-4 (neurotrophin 4), NT-5 (neurotrophin 5), NT-6 (neurotrophin 6), CNTF (ciliary neurotrophic factor), or GDNF (glia cell-derived neurotrophic factor);
32. The agent for improving energy metabolism according to the above 30 or 31, which is an agent for treating a patient of energy metabolic disorder;
33. The agent for improving energy metabolism according to any one of the above 30, 31 and 32, which is an agent for prevention of diseases induced by energy metabolic disorder;
34. The agent for improving energy metabolism according to the above 33, wherein the disease induced by energy metabolic disorder is non insulin dependent diabetes mellitus, insulin dependent diabetes mellitus, hyperlipemia, hypo-HDL-cholesterolemia, arteriosclerosis or hypertension;
35. The agent for improving energy metabolism according to the above 30 or 31, which ameliorates derangement of lipid metabolic disorder or carbohydrate metabolism;
36. The agent for improving energy metabolism according to the above 30 or 31, which is an agent for improving or activating energy metabolism of skeletal muscles or an agent for improving or activating energy metabolism of brown adipose cells;
37. An agent for improving physical constitution increasing energy production efficiency, which comprises as the active ingredient a neurotrophic factor;
38. An agent for improving thermogenesis, which comprises as the active ingredient a neurotrophic factor;
39. The agent for improving thermogenesis according to the above 38, which is an agent for improving cold constitution or hypothermia, an agent for inhibiting decreased body temperature in cold place, an agent for improving or normalizing decreased body temperature, or as an agent for prevention of frostbite;
40. The agent for improving thermogenesis according to the above 38 or 39, wherein the neurotrophic factor is NGF (nerve growth factor), BDNF (brain-derived neurotrophic factor), NT-3 (neurotrophin 3), NT-4 (neurotrophin 4), NT-5 (neurotrophin 5), NT-6 (neurotrophin 6), CNTF (ciliary neurotrophic factor), or GDNF (glia cell-derived neurotrophic factor);
41. The agent for improving energy metabolism or the agent for improving thermogenesis according to any one of the above 30, 31, 32, 33, 34, 35, 36, 37, 38 and 39, wherein the neurotrophic factor is BDNF;
42. The agent for improving energy metabolism or the agent for improving thermogenesis according to any one of the above 30, 31, 32, 33, 34, 35, 36, 37, 38 and 39, wherein the neurotrophic factor is NT-3;
43. The agent for improving energy metabolism or the agent for improving thermogenesis according to any one of the above 30, 31, 32, 33, 34, 35, 36, 37, 38 and 39, wherein the neurotrophic factor is NT-4/5;
44. The agent for improving energy metabolism or the agent for improving thermogenesis to any one of the above 30, 31, 32, 33, 34, 35, 36, 37, 38 and 39, wherein the neurotrophic factor is NT-6;
45. The agent for improving energy metabolism or the agent for improving thermogenesis according to any one of the above 30, 31, 32, 33, 34, 35, 36, 37, 38 and 39, wherein the neurotrophic factor is CNTF;
46. The agent for improving energy metabolism or the agent for improving thermogenesis according to any one of the above 30, 31, 32, 33, 34, 35, 36, 37, 38 and 39, wherein the neurotrophic factor is NGF;
47. The agent for improving energy metabolism or the agent for improving thermogenesis according to any one of the above 30, 31, 32, 33, 34, 35, 36, 37, 38 and 39, wherein the neurotrophic factor is GDNF;
48. The agent for improving energy metabolism agent or the agent for improving thermogenesis according to any one of the above 30, 31, 32, 33, 34, 35, 36, 37, 38 and 39, wherein the neurotrophic factor of the active ingredient is a trkA, a trkB and/or a trkC receptor agonist;

The meaning or definition of each term used in the present specification is explained below.

The "neurotrophic factor" means a physiologically active substance, which is secreted from the target cells for nerve growth, or by autocrine or paracrine, and promotes the growth, differentiation, or survival of neurons to form a neural circuit (synapse) in the living body. For example, the neurotrophic factor includes neurotrophins such as a nerve growth factor (hereinafter, abbreviated as NGF), a brain-derived neurotrophic factor (hereinafter, abbreviated as BDNF), a neurotrophin 3 (hereinafter, abbreviated as NT-3), a neurotrophin 4 (hereinafter, abbreviated as NT-4), a neurotrophin 5 (hereinafter, abbreviated as NT-5), and a neurotrophin 6 (hereinafter, abbreviated as NT-6); ciliary neurotrophic factor (hereinafter, abbreviated as CNTF); glia cell-derived neurotrophic factor (hereinafter, abbreviated as GDNF), etc. In addition, a modified recombinant neurotrophic factor produced by a substitution, a deletion, or an addition of a part of amino acid sequence of the naturally occurred neurotrophic factor sequence by a conventional technique may be included in the neurotrophic factor of the present specification, as far as it exhibits the similar physiological activity.

The "trkA, trkB and/or trkC receptor agonist" is a generic name for substances that are bound to trkA, trkB or trkC which is among the trk gene expression products being known as receptors for "neurotrophin", and activate them to exhibit their activities. Concretely, the known neurotrophin includes, for example, NGF binding to trkA, BDNF and NT-4 binding to trkB, and NT-3 binding to trkC, etc. This concept includes not only modified neurotrophins (modified by amino acid substitution, deletion or addition or sugar chain modification), but also peptides and organic compounds of a lower molecular weight as far as they exhibit a binding ability and activating ability to trkA, trkB or trkC receptor, for example, phosphorilating activity of tyrosine residue.

In the present specification, the "autonomic neuropathy" means dysfunction of sympathetic nervous system or parasympathetic nervous system. The autonomic neuropathy includes, for example, circulatory diseases (e.g., orthostatic hypotension (blackout), decreased ability to exercise, cardiac infarction sine dolore, angina pectoris sine dolore, etc.), gastrointestinal disorders (e.g., esophageal mobility abnormality, decreased gastric mobility (gastroparalysis), convulsion of pyloric portion of stomach, intestinal motility abnormality (diarrhea), decreased intestinal motility (constipation), decreased gallbladder contraction (cholecystitis), colon anus dysfunction (incontinence of feces), gluten sensitive enteropathy, etc.), urinary disorders (e.g., bladder diseases (atonic bladder, incontinence after urination, etc.), impotence, abnormal ejaculation, etc.), respiratory disorders (e.g., respiratory dysfunction, sleep apnea syndrome, etc.), thermoregulation disorders (e.g., sweat gland abnormality (perspiration abnormality), vasomotor abnormality (e.g., vasoconstriction, vasodilation disorder, nervous edema, etc.)), pupillary disorders (e.g., contracted pupil, mydriatic disorder, Argyll-Robertson syndrome, etc.), neuroendocrine disorders (e.g., pancreatic PP (polypeptide) hormonal secretion abnormality, somatostatin secretion abnormality, motilin, GIP secretion abnormality, gastrin secretion abnormality, norepinephrine secretion abnormality (uprise disorder), parathyroid hormone secretion abnormality, decreased atrial natriuretic peptide (ANP), asymptomatic hypoglycemia, etc.), etc. In addition to the above diseases, any autonomic neuropathy detectable by current various inspections for autonomic performance is included within the scope of the present invention. For example, autonomic neuropathies detectable by various inspections for sympathetic nerve performance such as the quantitative determination of norepinephrine in plasma/urine, the quantitative determination of dopamine-β-hydroxylase or c-AMP in plasma, thermal/pilocarpine sweating test, blood pressure measurement at standing (Schellong test), test for blood pressure rise under cooling (Heines-Brown test), norepinephrine-administration test, heart rate R-R oscillation can be included.

Autonomic neuropathy or autonomic imbalance is induced by causes such as diabetic mellitus, stress, menopausal symptoms, etc.

The "diabetic autonomic neuropathy" is a general name for autonomic neuropathy having as an underlying disease diabetes mellitus.

The "thermoregulation disorder" means a condition wherein body temperature cannot be normally controlled by abnormality of one of many thermoregulating functions which are mainly controlled by autonomic nervous system, for example, skin temperature sensor, thermoregulation center, metabolism of carbohydrate or lipid at the peripheral organs, skin vessel motion, perspiration function, etc. ([Thermoregulation and autonomic nerve]; Clinical Neurosci., vol. 14, no. 2, p. 128-129 (1996)). Representative disease thereof is "hypothermia" which is induced by diabetic ketoacidosis, hypoglycemia or hypothyroidism ([neuroendocrine abnormalities and body temperature], Rinsho Kango (i.e., clinical nursing), vol. 8, no. 14, p. 2202-2206 (1982)). The "body temperature" in the present specification includes skin temperature as well ([coldness in hands and feet, dyshidrosis (autonomic nervous tension)], Shindan To Chiryo (i.e., diagnosis and treatment), vol. 71, no. 2, p. 262-266).

The "action of inhibiting norepinephrine content decrease in peripheral tissue" means an action of increasing norepinephrine content in tissue of a patient wherein the norepinephrine content is decreased in the peripheral organs such as muscles and heart. This action can be clinically monitored by the quantitative determination of norepinephrine in serum/urine, or by the quantitative determination of dopamine-β-hydroxylase or c-AMP in plasma.

The "agent for improving decreased peripheral organ functions" means an action of activating or normalizing the metabolism of the peripheral organs such as muscles or brown adipose tissue of a patient of autonomic neuropathy, whose energy metabolism level is lowered than the normal level, or whose thermogenesis is lowered. This action can be evaluated by the determination of oxygen consumption or the thermometry under fasting.

The "agent for improving energy metabolism" means an agent for controlling the relation between the energy intake and the energy consumption and adjusting it to the normal conditions or normalizing it, in a patient having an abnormality of the relation between the energy intake and the energy consumption, or who cannot normally control the relation between the energy intake and the energy consumption. In other words, it means an agent for adjusting a condition under energy metabolic disorder or energy metabolism dysfunction to the normal condition or normalizing it.

The "agent for improving thermogenesis" means an agent for adjusting a condition under thermogenesis disorder or thermogenesis dysfunction to the normal condition or normalizing it, an agent for adjusting a condition of hypothermia to the normal condition or normalizing it, or an agent for adjusting a condition of hypothermia induced by external causes such as cold climates or cold place to the normal condition or normalizing it.

### BRIEF DESCRIPTION OF THE DRAWINGS

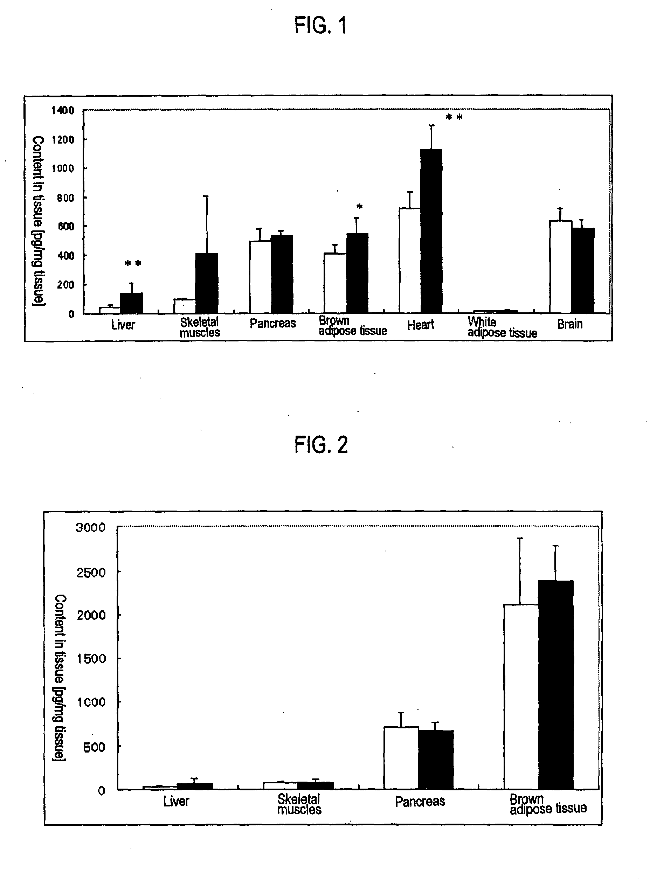

Fig. 1 shows the effects of BDNF administration on the norepinephrine content in tissue of diabetic mice. BDNF was subcutaneously administered at a dose of 20 mg/kg every day to *db/db* mice (♂, 12-weeks old when administration began). Two weeks after the administration, each tissue was excised and the norepinephrine content in tissue was determined by high performance chromatograph system (HPLC). The data are expressed by average ± SD, n= 6-7. The * and ** mean that there are significant differences of P<0.05, P<0.01 in Student's t-test, respectively. □: vehicle-treated group (vehicle alone, hereinafter the same), ■: BDNF-treated group.

Fig. 2 shows the effects of BDNF administration on the norepinephrine content in tissue of normal mice. BDNF was subcutaneously administered at a dose of 20 mg/kg every day to BALB mice (♂, 10-weeks old when administration began). Two weeks after the administration, each tissue was excised and the norepinephrine content in tissue was determined by HPLC. The data are expressed by average ± SD, n= 6-7. □: vehicle-treated group, and ■: BDNF-treated group.

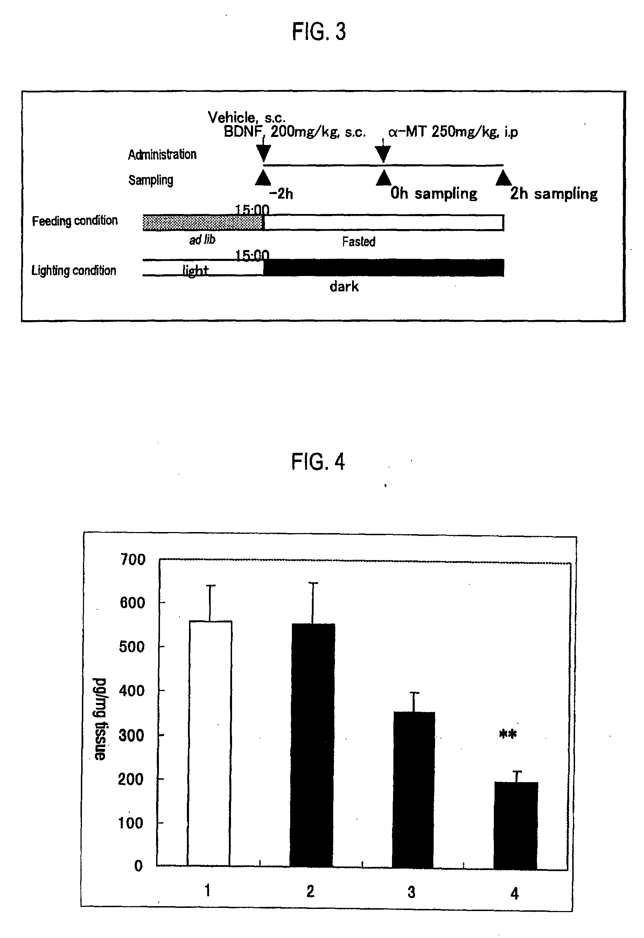

Fig. 3 shows the experimental schedule of BDNF single administration on norepinephrine turnover in *db/db* mice.

Fig. 4 shows the effects of BDNF on norepinephrine turnover in brown adipose tissue of *db/db* mice. To *db/db* mice (♂, 12-weeks old), BDNF was subcutaneously administered at a dose of 200 mg/kg in Group 2 and Group 4, while the vehicle was subcutaneously administered instead in Groups 1 and 3. Two hours after the administration, the tissues were excised in Groups 1 and 2. In Groups 3 and 4, α-MT was administered intraperitoneally, and two hours thereafter, the tissues were excised. The NE content in the excised tissues was determined by HPLC. 1: vehicle-treated group; 2: BDNF-treated group; 3: (vehicle + α-MT)-treated group; 4: (BDNF + α-MT)-treated group. The data are expressed by average ± SD, n= 7. There was no significant difference between Group 1 and Group 2 in each tissue. The ** means that there is a significant difference of P<0.01 in Student's t-test, as compared with Group 3 ((vehicle + α-MT)-treated group).

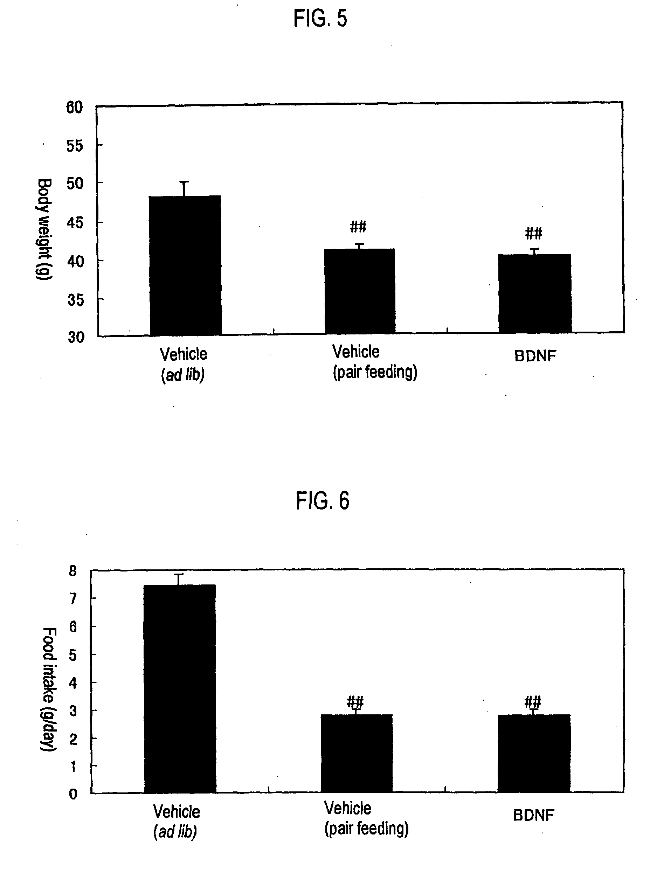

Fig. 5 shows the effects of BDNF administration on body weight of diabetic mice. The abscissa axis indicates each Group, and the axis of ordinate indicates body weight (g). The columns and the error bars indicate the average body weight and the standard deviation (n=7), respectively. The ## means that there is a significant difference of P<0.01 in Turkey's test, as compared with the vehicle-treated group (*ad libitum*).

Fig. 6 shows the effects of BDNF administration on food intake in diabetic mice. The abscissa axis indicates each Group, and the axis of ordinate indicates food intake (g/day). The columns and the error bars indicate the average body weight and the standard deviation (n=7), respectively. The ## means that there is a significant difference of P<0.01 in Turkey's test, as compared with the vehicle-treated group (*ad libitum*).

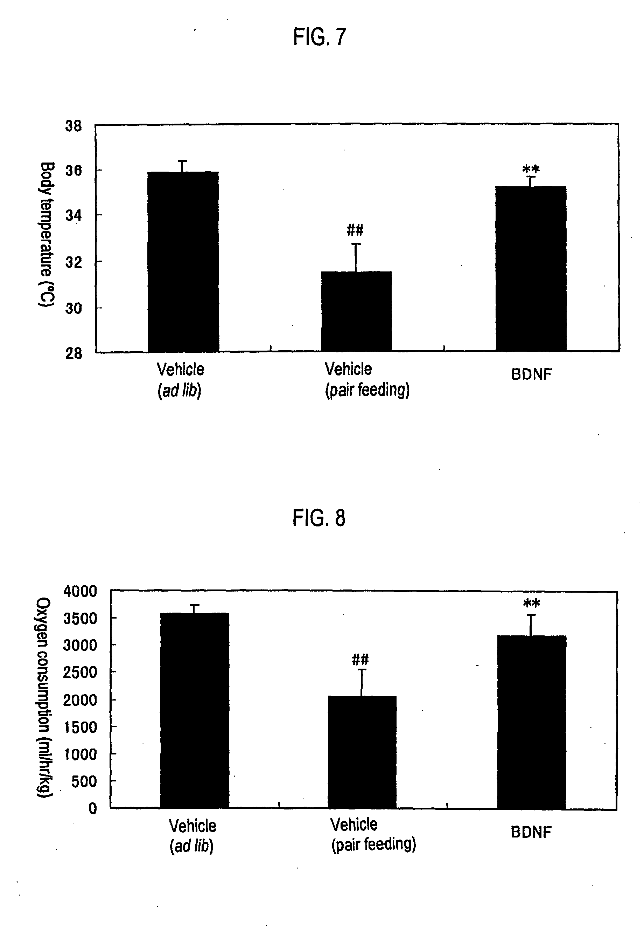

Fig. 7 shows the effects of BDNF administration on body temperature of diabetic mice. The abscissa axis indicates each Group, and the axis of ordinate indicates body temperature (°C). The columns and the error bars indicate the average body weight and the standard deviation (n=7), respectively. The ## means that there is a significant difference of P<0.01 in Turkey's test, as compared with the vehicle-treated group (*ad libitum*). The ** means that there is a significant difference of P<0.01 in Turkey's test, as compared with the vehicle-treated group (pair feeding).

Fig. 8 shows the effects of BDNF administration on oxygen consumption in diabetic mice. The abscissa axis indicates each Group, and the axis of ordinate indicates oxygen consumption (ml/hr/kg). The columns and the error bars indicate the average body weight and the standard deviation (n=7), respectively. The ## means that there is a significant difference of P<0.01 in Turkey's test, as compared with the vehicle-treated group (*ad libitum*). The ** means that there is a significant difference of P<0.01 in Turkey's test, as compared with the vehicle-treated group (pair feeding)

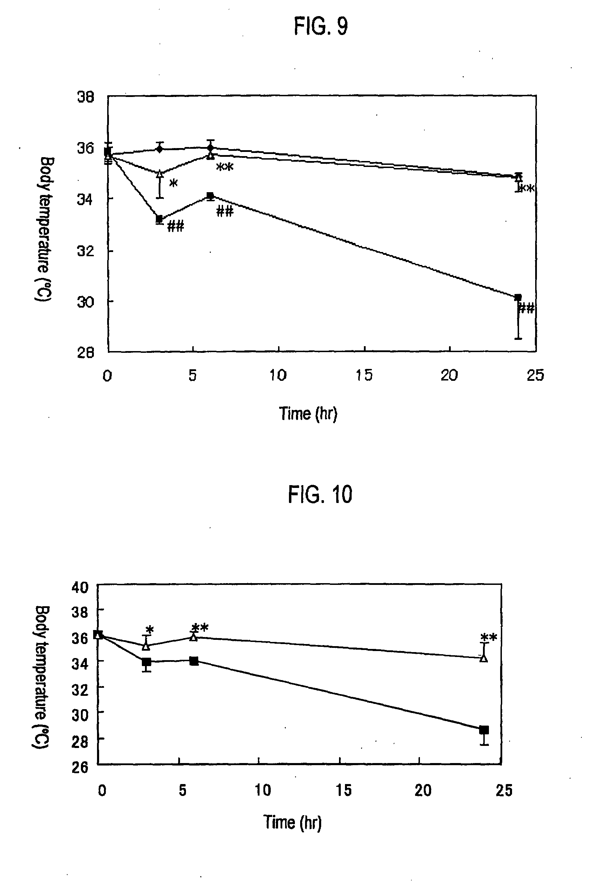

Fig. 9 shows the effects of BDNF single administration (125 mg/kg) on body temperature of diabetic mice. The abscissa axis indicates time after BDNF administration, and the axis of ordinate indicates body temperature (°C). ◆ means the average body temperature at each point in the vehicle-treated group (*ad libitum,* n=3), ■ means the average body temperature at each point in the vehicle-treated group (fasted, n=3), and Δ means the average body temperature at each point in the BDNF-treated group (fasted, n=3), respectively. The error bars indicate the standard deviations at each point. The ## means that there is a significant difference of P<0.01 in Turkey's test, as compared with the vehicle-treated group (*ad libitum).* The * and ** mean that there are significant differences of P<0.05 and P<0.01 in Turkey's test, respectively, as compared with the vehicle-treated group (fasted).

Fig. 10 shows the effects of BDNF single administration (20 mg/kg) on body temperature of diabetic mice. The abscissa axis indicates time after BDNF administration, and the axis of ordinate indicates body temperature (°C). ■ means the average body temperature at each point in the vehicle-treated group (fasted, n=7), and Δ means the average body temperature at each point in the BDNF-treated group (fasted, n=7), respectively. The error bars indicate the standard deviations at each point. The * and ** mean that there are significant differences of P<0.05 and P<0.01 in Student's t-test, respectively, as compared with the vehicle-treated group (fasted).

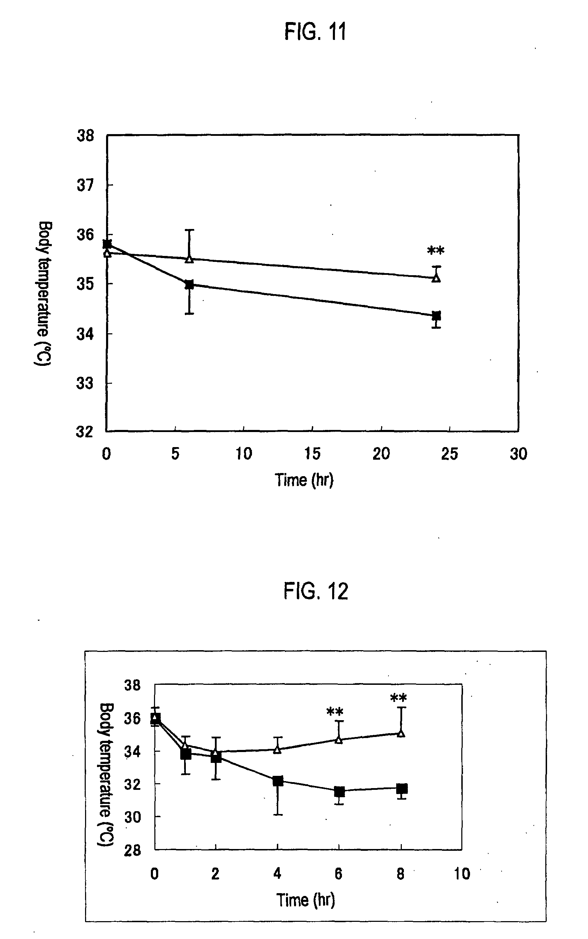

Fig. 11 shows the effects of BDNF administration on body temperature of type 1 diabetic mice. The abscissa axis indicates time after BDNF administration, and the axis of ordinate indicates body temperature (°C). ■ means the average body temperature (°C) at each point in the vehicle-treated group (fasted, n=4), and Δ means the average body temperature (°C) at each point in the BDNF-treated group (fasted, n=4), respectively. The ** means that there is a significant difference of P<0.01 in Student's t-test, as compared with the vehicle-treated group.

Fig. 12 shows the effects of BDNF administration on body temperature of cold-exposed mice. The abscissa axis indicates time after BDNF administration, and the axis of ordinate indicates body temperature (°C). ■ means the average body temperature (°C) at each point in the vehicle-treated group (n=5), and Δ means the average body temperature (°C) at each point in the BDNF-treated group (n=5), respectively. The ** means that there is a significant difference of P<0.01 in Student's t-test, as compared with the vehicle-treated group.

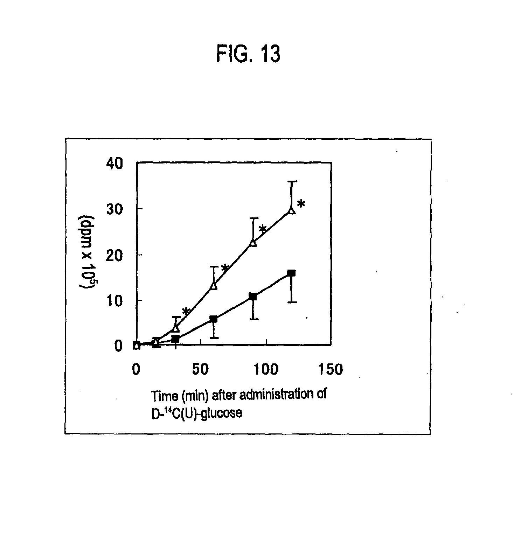

Fig. 13 shows the action of BDNF on carbohydrate metabolism of *db/db* mice. The abscissa axis indicates time after D-[U-¹⁴C]-glucose administration, and the axis of ordinate indicates ¹⁴C amount as released as CO₂ (dpm x 10⁵). ■ means the ¹⁴C amount as released as CO₂ (dpm x 10⁵) at each point in the vehicle-treated group (n=5), and Δ means the ¹⁴C amount as released as CO₂ (dpm x 10⁵) at each point in the BDNF-treated group (n=5), respectively. The * means that there is a significant difference of P<0.05 in Student's t-test, as compared with the vehicle-treated group.

### BEST MODE FOR CARRYING OUT THE INVENTION

The neurotrophic factors used as an active ingredient of the present invention may be commercially available ones or can be prepared by the following methods.

The neurotrophic factors used as the active ingredient of the present invention can be any ones prepared by various methods as far as they are purified to such a degree that it could be used as a medicament. The neurotrophic factor can be obtained by cultivating a primary culture cell or an established cell line that can produce the neurotrophic factor, and isolating and purifying it from the culture medium thereof (e.g., culture supernatant, cultured cells). Moreover, a recombinant neurotrophic factor can be obtained by a conventional gene engineering technique, e.g., by inserting a gene encoding a neurotrophic factor into a suitable vector, transforming a suitable host with the recombinant vector, and isolating from a culture supernatant of the resulting transformant. The host cells to be used in the above process are not limited, and may be any conventional host cells which have been used in a gene engineering technique, for example, *Escherichia coli*, *Bacillus subtilis*, yeasts, mold fungi, plant cells or animal cells.

The neurotrophic factors obtained in the above method include a modified recombinant neurotrophic factor such as ones produced by a deletion of a part of amino acid sequence, or a substitution by other amino acid(s), or an addition of a part of other amino acid sequence, or ones having one or more amino acids at the N-terminus and/or the C-terminus, or ones wherein the sugar chain is deleted or substituted, as far as they exhibit substantially the same activity.

### Method for Preparation of BDNF:

When a conventional gene engineering technique is employed, BDNF is prepared by inserting a gene encoding BDNF into a suitable vector, transforming a suitable host with the recombinant vector, and isolating it from a culture supernatant of the resulting transformant (cf., Proc. Natl. Acad. Sci. USA, vol. 88, p. 961 (1991); Biochem. Biophys. Res. Commun., vol. 186, p. 1553 (1992)). The gene engineering technique is suitable for production of BDNF of same quality in a large scale. The host cells mentioned above are not limited, but may be any conventional host cells which have been used in a gene engineering technique, for example, *Escherichia coli*, *Bacillus subtilis*, yeasts, plant cells or animal cells.

### Method for preparation of NT-3:

NT-3 can be prepared by express in various host cells in the same manner as in the preparation of BDNF. The methods for preparing thereof and the methods for assay thereof are disclosed in Neuron, vol. 4, 767-773 (1990), or JP-A-5-161493 (WO 91/3659).

### METHOD FOR PREPARATION OF NT-4:

NT-4 can be prepared by express in various host cells in the same manner as in the preparation of BDNF. The methods for expression of the recombinant NT-4 and the methods for assay thereof are disclosed in Proc. Natl. Acad. Sci. USA, vol. 89, p. 3060-3064 (1992.4), JP-A-7-509600 (WO 93/25684), or JP-A-6-501617 (WO 92/5254).

### METHOD FOR PREPARATION OF CNTF:

CNTF can be prepared in a large scale by expressing in various host cells in the same manner as in the preparation of BDNF. The methods for expression of the recombinant CNTF and the methods for assay thereof are disclosed in Biochimica et Biophysica Acta, vol. 1090, p. 70-80 (1991), J. Neurochemistry, vol. 57, p. 1003-1012 (1991). The methods for preparing the recombinant CNTF and the purification thereof are disclosed in JP-A-4-502916 (WO 90/7341).

The agent for treatment of autonomic neuropathy, agent for treatment or prevention of autonomic imbalance, agent for improving energy metabolism, and agent for improving thermogenesis of the present invention, which comprise as the active ingredient a neurotrophic factor, can be administered either parenterally or orally.

The precise dosage and the administration schedule of the above agents of the present invention should vary according to the dosage to be required for each patient, the method for treatment, the disease to be treated, or the degree of necessity, and further according to the diagnosis by a physician. When administered parenterally, the dosage and the frequency of the administration may vary according to the conditions, ages, body weights of patients, and administration routes, but when it is administered subcutaneously or intravenously in the form of an injection, then the daily dosage thereof is in the range of about 1 to about 2500 µg, preferably in the range of about 10 to about 500 µg per 1 kg of the body weight in an adult. When it is administered to the air tract in the form of an aerosol spray, the daily dosage thereof is in the range of about 1 µg to about 2500 µg, preferably in the range of about 10 to about 500 µg per 1 kg of the body weight in an adult. The administration schedule is either continuous daily administration, intermittent administration, or a schedule of combining these methods. When administered orally, the dosage and the frequency of administration may vary according to the conditions, ages, body weights of patients, and administration routes, and the daily dosage thereof is in the range of about 5 to about 2500 µg, preferably in the range of about 10 to about 1000 µg per 1 kg of the body weight in an adult.

A pharmaceutical composition can be prepared by mixing a neurotrophic factor with a pharmaceutically acceptable non-toxic carrier. When a pharmaceutical composition for parenteral administration (subcutaneous injection, intramuscular injection, or intravenous injection) is prepared, it is preferably in the form of a solution preparation or a suspension preparation. When a pharmaceutical composition for intravaginal administration or rectal administration is prepared, it is preferably in the form of a semi-solid preparation such as cream or suppository. When a pharmaceutical composition for intranasal administration is prepared, it is preferably in the form of a powder, a nasal drop, or an aerosol.

The pharmaceutical composition is administered in the form of a single dosage unit, and can be prepared by any conventional method that is known in the pharmaceutical field such as methods disclosed in Remington's Pharmaceutical Science (published by Mack Publishing Company, Easton, PA, 1970). An injection preparation may optionally contain as a pharmaceutical carrier a protein derived from plasma such as albumin, an amino acid such as glycine, or a carbohydrate such as mannitol, and additionally a buffering agent, a solubilizer, or an isotonic agent, etc. can be contained. When the present pharmaceutical composition is in the form of an aqueous solution preparation or a lyophilized preparation, it may preferably contain a surfactant such as Tween 80 (registered trade mark), Tween 20 (registered trade mark), etc. in order to avoid aggregation. When the present pharmaceutical composition is a composition for parenteral administration other than an injection preparation, then it may contain distilled water or physiological saline solution, polyalkylene glycol such as polyethylene glycol, an oil derived from plant, hydrogenated naphthalene, etc. For example, a pharmaceutical composition such as a suppository for intravaginal administration or rectal administration may contain as a conventional excipient polyalkylene glycol, vaseline, cacao butter, etc. A pharmaceutical composition for intravaginal administration may contain an absorbefacient such as a bile salt, an ethylenediamine salt, a citrate, etc. A pharmaceutical composition for inhalation may be in the form of a solid preparation, and may contain as an excipient lactose, etc., and a pharmaceutical composition for intranasal drop may be in the form of an aqueous solution or an oily solution.

The present pharmaceutical composition is especially preferable in the form of a formulation by which the present compound can persistently be given to a subject by a single administration for a long term, e.g., for one week or one year, and various sustained release preparations, depot preparations, or implant preparations can be employed. For example, a pharmaceutical composition may contain a neurotrophic factor per se, or a pharmaceutically acceptable salt of a neurotrophic factor of which solubility in body fluid is low. Such pharmaceutically acceptable salts are, for example, (1): an acid addition salt such as phosphate, sulfate, citrate, tartrate, tannate, pamoate, alginate, polyglutarate, naphthalenemono- or di-sulfonate, polygalacturonate, etc., (2): a salt or complex with polyvalent metal cation such as zinc, calcium, bismuth, barium, nickel, etc, or a combination of (1) and (2), for example, a tannic acid zinc salt, etc. A neurotrophic factor is preferably converted into a slightly-water-soluble salt thereof, which is mixed with a gel, for example, aluminum monostearate gel and sesame oil, etc. to give a suitable injection preparation. In this case, especially preferable salt is a zinc salt, a tannic acid zinc salt, a pamoate, etc. Another type of a sustained release injection preparation is ones wherein a neurotrophic factor is preferably converted into a slightly-water-soluble salt thereof, which is further enclosed in a slow-disintegrative non-toxic and non-antigenic polymer such as a polymer or a copolymer of polylactic acid/polyglycolic acid. In this case, especially preferable salt is zinc salt, tannic acid zinc salt, pamoate, etc. In addition, a neurotrophic factor or a slightly-water-soluble salt thereof can be enclosed into a cholesterol matrix or collagen matrix to give a sustained release preparation.

The pharmaceutical preparation for oral administration may be ones which are prepared by microencapsulating a neurotrophic factor or a salt thereof with lecithin, cholesterol, a free fatty acid, or ones which are prepared by enclosing said microcapsules into gelatin capsules, or ones which are prepared by enclosing a neurotrophic factor or a salt thereof in enteric capsules, etc. These preparations may additionally contain, for example, an absorbefacient, a stabilizer, a surfactant, etc.

### (Toxicity)

When a neurotrophin, especially BDNF, was administered subcutaneously to rats and cynomolgus monkeys at a dose of 100 mg/kg and 60 mg/kg, respectively, for four weeks, no animal died. With respect to the acute toxicity, BDNF was administered to rats and cynomolgus monkeys at a dose of 200 mg/kg, and no animal died. Therefore, BDNF shows high safety.

The present invention is illustrated by Examples.

### Example 1: Effects of BDNF administration of norepinephrine content in tissue

### (1) Materials and method for experiment

Reagents: BDNF was purchased from REGENERON PHARMACEUTICALS, INC., and used. Commercially available D-PBS (manufactured by GIBCO, phosphate buffered saline solution) was purchased and used. The other reagents were commercially available ones with the best quality.

Test animals: Male C57 BL/Ksj-*db/db* mice (SPF standard) were purchased from Clea Japan, Inc. as an animal model for disease. After pre-feeding, the animals were used in the experiment at 12 weeks old. As a normal animal model, male BALB/c mice (SPF) were purchased from Charles River Japan, Inc., and after pre-feeding, they were used in the experiment at 10 weeks old.

Breeding conditions: The mice were kept in a room controlled at a temperature of 23±2°C under a humidity of 55±10 %, with an illumination cycle of light on (8:00 to 20:00) and light off (20:00 to 8:00). During the pre-feeding and the experiment, the animals were given food (CE-2, Clea Japan, Inc.) and sterilized tap water *ad libitum.*

### (2) Preparation of dosing solution

A BDNF solution was prepared by diluting with D-PBS (Gibco) to a concentration of 2.0 mg/ml, and used in the experiment.

### (3) Measurement of norepinephrine content

The norepinephrine content in each tissue was determined by using HPLC (Eicom Corporation).

### (4) Effects of BDNF administration on norepinephrine content in tissue of animal models for autonomic neuropathy

*db/db* Mice (male, 12 weeks old when administration began) were grouped into two groups with respect to the blood glucose levels and the body weights thereof, and to each group, D-PBS (vehicle) alone or BDNF (20 mg/kg) was subcutaneously administered daily (number of test animals; vehicle-treated group: n=6, BDNF-treated group: n=7). Two weeks after the administration, the animals were put down by cervical dislocation, and each tissue was excised from the mice, and cathecholamine therein was extracted with a perchloric acid solution. The norepinephrine content in the extract was determined by HPLC, and divided by the weight of the excised tissue, and from which the norepinephrine content in tissue was calculated. The results are shown in Fig. 1. There were significant increases in the norepinephrine content in brown adipose tissue, heart and liver in the BDNF-treated group, as compared with those in the vehicle-treated group.

### (5) Effects of BDNF administration on norepinephrine content in tissue in normal animals

Under the same conditions for BDNF administration as the above (4), the norepinephrine content in each tissue of BALB/c mice was examined. The results are shown in Fig. 2. There was no difference of the norepinephrine contents between both Groups either in the brown adipose tissue, liver, pancreas or skeletal muscle. Since norepinephrine is a transmitter in the sympathetic nervous system, it was indicated from the results of the above (4) and (5) that BDNF may activate the sympathetic nervous system only in the animals having autonomic neuropathy, especially having depressed sympathetic nerve function.

### Example 2: Effects of BDNF single administration on norepinephrine turnover in db/db mice

### (1) Materials and method for experiment

Reagents: BDNF was purchased from REGENERON PHARMACEUTICALS, INC., and used. The other reagents were commercially available ones with the best quality.

Test animals: Male C57 BL/Ksj-*db/db* mice (SPF standard) were purchased from Clea Japan, Inc. as an animal model for disease. After pre-feeding, the animals were used in the experiment at 12 weeks old.

Breeding conditions: The mice were kept in a room controlled at a temperature of more than 20°C but below 26°C, under a relative humidity of more than 30 % but below 70 %, with an illumination cycle of light on (3:00 to 15:00) and light off (15:00 to 3:00). During the pre-feeding and the experiment, the animals were given food (CE-2, Clea Japan, Inc.) and sterilized tap water *ad libitum.*

### (2) Preparation of dosing solution

A BDNF solution was prepared by diluting with the following phosphate buffer (vehicle) to a concentration of 20 mg/ml, and used in the experiment. The vehicle is a phosphate buffer (10 mM phosphoric acid, 150 mM sodium chloride, pH 7.0±0.2) containing 1 % mannitol and 0.01 % Tween 80.

### (3) Measurement of norepinephrine content

The norepinephrine content in the extract from each tissue was determined by HPLC (Eicom Corporation).

### (4) Effects of BDNF single administration on norepinephrine turnover

*db/db* Mice (male, 12 weeks old when administration began) were grouped into four groups so that the glycosylated hemoglobin (HbAlc) levels and the body weights were not biased in each group. On the following day, when the dark time started, the vehicle was subcutaneously administered to Group 1 and Group 3, and BDNF (200 mg/kg) was subcutaneously administered to Group 2 and Group 4. The food was removed immediately after the administration, and thereafter, the animals were kept fasted. Two hours after the administration, the animals in Groups 1 and 2 were decapitated, and the brown adipose tissues were excised. To the remaining two Groups, α-MT, which is an NE synthesis inhibitor, was intraperitoneally administered at a dose of 250 mg/kg, and two hours after the α-MT administration, the animals in these Groups were decapitated, and the tissues were excised therefrom. The tissues thus excised were rapidly lyophilized in a liquid nitrogen, and kept at -80°C until extraction. Cathecholamine was extracted with a perchloric acid solution, and the norepinephrine content in the extract was determined by HPLC, and divided by the weight of the excised tissue, and from which the norepinephrine content in tissue was calculated. The group construction and the experimental schedule were shown in Table 1 and Fig. 3.

**Table 1**

| Group No. (Animal No.) | Group 1 (11-17) | Group 2 (21-27) | Group 3 (31-37) | Group 4 (41-47) |
|---|---|---|---|---|
| Dosage of test substance (mg/kg) | 0 | 200 | 0 | 200 |
| Dosage of α-MT (mg/kg) | 0 | 0 | 250 | 250 |
| Time of excitation of tissue (time (h) after α-MT admin.) | 0 | 0 | 2 | 2 |
| Number of animals | 7 | 7 | 7 | 7 |
| Concentration of dosing solution of test substance (mg/ml) | 0 | 20 | 0 | 20 |
| Volume of dosing solution of test substance (ml/kg) | 10 | 10 | 10 | 10 |
| Concentration of dosing solution of α-MT (mg/ml) | - | - | 25 | 25 |
| Volume of dosing solution of α-MT (ml/kg) | - | - | 10 | 10 |
| *: Test compound (0 mg/kg) means that only the vehicle was administered, and α-MT (0 mg/kg) means that α-MT was not administered at all. | | | | |

The results are shown in Fig. 4. When the data of Group 1 and Group 2 were studied, there was no significant difference in the norepinephrine content at two hours after the BDNF administration (i.e., before α-MT administration) as compared with that of the vehicle-treated group. When the data of Group 3 and Group 4 were compared with the data of Group 1 and 2, the NE content was lowered two hours after α-MT administration, as compared with the data before α-MT administration. It means that the synthesis of norepinephrine was inhibited by α-MT. Under these conditions, there was observed a significant decrease in the norepinephrine content in the BDNF-treated group, as compared with the vehicle-treated group. From these results, it was indicated that BDNF increases the norepinephrine turnover in the brown adipose tissue. By this fact, it was confirmed that BDNF accelerates the sympathetic nerve activity projected from the central system to the brown adipose tissue in *db/db* mice.

### Example 3: Effects of BDNF administration on regulation of body temperature, thermogenesis and energy metabolism

### (1) Materials and method for experiment

Reagents: BDNF was purchased from REGENERON PHARMACEUTICALS, INC., and used. The other reagents were commercially available ones with the best quality.

Test animals: Male C57 BL/Ksj-*db/db* mice (SPF standard) were purchased from Clea Japan, Inc. After pre-feeding for two weeks, the animals were used in the experiment at 12 weeks old. The mice were kept in a room controlled at a temperature of 23±2°C under a humidity of 55±10 %, with an illumination cycle of light on (8:00 to 20:00) and light off (20:00 to 8:00). During the pre-feeding, the animals were given food (CE-2, Clea Japan, Inc.) and sterilized tap water *ad libitum,* but if necessary, they were fasted when used in the experiment.

### (2) Preparation of BDNF dosing solution

A BDNF solution was prepared by diluting with the following phosphate buffer (vehicle) to a concentration of 20 mg/ml, and used in the experiment. The vehicle was a phosphate buffer (10 mM phosphoric acid, 150 mM sodium chloride, pH 7.0±0.2) containing 1 % mannitol and 0.01 % Tween 80.

### (3) Method for measuring body temperature and statistics analysis thereof

Body temperature was measured with a rectal thermometer (manufactured by Physitemp Instruments Inc., Model BAT 12).

The data thereof were analyzed by Turkey's test or Student's t-test.

### (4) Method for measurement of oxygen consumption and statistics analysis thereof

Oxygen consumption was measured with using a metabolism measuring system for small animals (manufactured by Muromachi Kikai Co., Ltd., Model MK-5000). Concretely, the mice were put into an airtight chamber for about 3 hours, and then thereto was blown air at a flow rate of 180 ml/min, and the reduction in oxygen concentration was monitored. The oxygen consumption when the mice were keeping quiet was employed as a test result. Each measurement was analyzed by Turkey's test.

### (5) Measurement of glycosylated hemoglobin (HbAlc)

The blood was taken from the tail vein of the mice, and the glycosylated hemoglobin therein was measured by DCA-2000 (Sankyo Bayer, Ltd.).

### (6) Measurement of blood glucose level

The blood was taken from the tail vein of the mice, and the blood glucose level was measured with Glucose CII Test Wako (manufactured by Wako Pure Chemical Industries, Ltd.).

### (7) Method for pair feeding

Using a synchronized pair-feeding apparatus, the food intake in both the BDNF-treated group and the vehicle-treated were controlled so as to be the same.

### (8) Effect of BDNF daily administration on thermogenesis and energy metabolism

*db/db* Mice were grouped into three groups so that the HbAlc and the body weights were not biased in each group (7 animals/group). Group 1: vehicle-treated group (*ad libitum*), Group 2: vehicle-treated group (pair feeding) (only the vehicle in the same amount as BDNF-treated group was administered), and Group 3: BDNF (20 mg/kg)-treated group. BDNF was subcutaneously administered daily at a dose of 20 mg/kg, and on the 13th day, the body weight, the food intake and the body temperature were measured. The results are shown in Fig. 5, Fig. 6 and Fig. 7, respectively. As compared with the vehicle-treated group in which the animals were pair-fed in the same amount as the BDNF-treated group, there was no change in the body weight in the BDNF-treated group, but the decreased body temperature found in the vehicle-treated group was significantly inhibited in the BDNF-treated group. From this fact, it was assumed that BDNF increases energy metabolism, and hence, as an index for basal metabolism, the oxygen consumption when the animals were keeping quiet was measured on the 15th to the 18th day. The results are shown in Fig. 8. As compared with the vehicle-treated group with pair-feeding, there was a significant increase in the oxygen consumption in the BDNF-treated group. From these data, when compared with the mice to which food intake was controlled to the same, the thermogenesis and the basal metabolism can be promoted by BDNF administration.

### (9) Effect of BDNF single administration on thermogenesis

*db/db* Mice were grouped into three groups so that the blood glucose levels and the body weights were not biased in each group. Group 1: vehicle-treated group (*ad libitum),* Group 2: vehicle-treated group (fasted), (immediately after the administration of the vehicle in the same amount as BDNF-treated group, the food was removed and thereafter, the animals were kept fasted), and Group 3: BDNF-treated group (fasted) (after BDNF administration, the animals were kept fasted in the same manner as the above). BDNF was subcutaneously administered once at a dose of 125 mg/kg (3 animals/group) or at a dose of 20 mg/kg (7 animals/group). The body temperature was measured at 0, 3, 6, and 24 hours thereafter. These results are shown in Fig. 9 and Fig. 10, respectively. Fig. 9 shows the data of the BDNF (125 mg/kg)-treated group (-Δ-), the vehicle-treated group (free fed) (-●-), and the vehicle-treated group (fasted) (-■-). Fig. 10 shows the data of the BDNF (20 mg/kg)-treated group (-Δ-), and the vehicle-treated group (fasted) (-■-) (no data of the vehicle-treated group (free fed)). From these results, the decreased body temperature found in the vehicle-treated group was significantly inhibited in the BDNF-treated group under fasting, as compared with the vehicle-treated group. It was confirmed that the effect of BDNF on regulation of thermogenesis was rapid and exhibited within 24 hours after the BDNF-administration.

### Example 4: Effects of BDNF on thermogenesis of type 1 diabetic animal

### (1) Materials and method for experiment

Reagents: BDNF was purchased from REGENERON PHARMACEUTICALS, INC., and used. The other reagents were commercially available ones with the best quality.

Test animals: Male Wistar rats (SPF standard) were purchased from Charles River Japan Inc. After pre-feeding, the animals were used in the experiment at 10 weeks old. The animals were kept in a room controlled at a temperature of 23±2°C under a humidity of 55±10 %, with an illumination cycle of light on (8:00 to 20:00) and light off (20:00 to 8:00). During the pre-feeding, the animals were given food (CE-2, Clea Japan, Inc.) and sterilized tap water *ad libitum*, but if necessary, they were fasted when used in the experiment.

### (2) Preparation of dosing solutions of streptozotocin (STZ) and BDNF

STZ was dissolved in 10 mM sodium citrate solution (pH 5.5) to give a 32.5 mg/ml solution. The STZ solution was prepared when used, and used within 5 minutes after dissolution. A BDNF solution was prepared by diluting with the following phosphate buffer (vehicle) to a concentration of 20 mg/ml, and used in the experiment. The vehicle was a phosphate buffer (10 mM phosphoric acid, 150 mM sodium chloride, pH 7.0±0.2) containing 1 % mannitol and 0.01 % Tween 80.

### (3) Method for measuring body temperature and statistic analysis thereof

Body temperature was measured with a rectal thermometer (manufactured by Physitemp Instruments Inc., Model BAT 12).

The data thereof were analyzed by Student's t-test.

### (4) Induction of STZ-induced diabetes and selection of hyperglycemic animals

To the pre-fed rats was administered intraperitoneally the STZ solution at a ratio of 2 ml/kg (at a dose of STZ 65 mg/kg). Five days after the STZ administration, the blood glucose level was measured, and according to the results thereof, the rats showing no hyperglycemia (300 mg/dl or below), and the rats apparently being in bad conditions were excluded from the experiment.

### (5) Effect of BDNF single administration on thermogenesis

The STZ-induced diabetic rats were grouped into two groups at 5 weeks after the STZ-administration so that the body weights and the body temperatures were not biased in each group (4 animals/group). Group 1: vehicle-treated group, and Group 2: BDNF-treated group. BDNF was administered subcutaneously once at a dose of 20 mg/kg, and after the administration, the animals were kept fasted. The body temperature at 0, 6, 24 hours after the administration was measured. The results are shown in Fig. 11 (in the figure, -Δ-: BDNF-treated group; -■-: vehicle-treated group).

As is shown in Fig. 11, the decreased body temperature found in the vehicle-treated group was significantly inhibited in the BDNF-treated group under fasting, as compared with the vehicle-treated group. It was confirmed that BDNF's action on regulation of thermogenesis was effective not only to the obesity diabetic animals (*db/db* mice) with hyperinsulinemia but also to the non-obesity type 1 diabetic animals with insulin deficiency.

### Example 5: Inhibitory effect of BDNF against decreased body temperature of cold-exposed mice

### (1) Materials and method for experiment

Reagents: BDNF was purchased from REGENERON PHARMACEUTICALS, INC., and used. The other reagents were commercially available ones with the best quality.

Test animals: Male C57 BL/Ksj*-db/db* mice (SPF standard) were purchased from Clea Japan, Inc. After pre-feeding for two weeks, the animals were used in the experiment at 12 weeks old. The mice were kept in a room controlled at a temperature of 23±2°C under a humidity of 55±10 %, with an illumination cycle of light on (8:00 to 20:00) and light off (20:00 to 8:00). During the pre-feeding, the animals were given food (CE-2, Clea Japan, Inc.) and sterilized tap water *ad libitum,* but if necessary, they were fasted when used in the experiment.

### (2) Preparation of dosing solution

A BDNF solution was prepared by diluting with the following phosphate buffer (vehicle) to a concentration of 20 mg/ml, and used in the experiment. The vehicle was a phosphate buffer (10 mM phosphoric acid, 150 mM sodium chloride, pH 7.0±0.2) containing 1 % mannitol and 0.01 % Tween 80.

### (3) Method for measuring body temperature and statistics analysis thereof

Body temperature was measured with a rectal thermometer (manufactured by Physitemp Instruments Inc., Model BAT 12).

The data thereof were analyzed by Student's t-test.

### (4) Inhibitory effect of BDNF against decreased body temperature of cold-exposed mice

*db/db* Mice were grouped into two groups with respect to the body weights thereof (5 animals/group). Group 1: vehicle-treated group, and Group 2: BDNF-treated group. BDNF was subcutaneously administered at a dose of 20 mg/kg once, and after the administration, the animals were kept in a cage and put in a constant temperature bath under cold condition (15°C), during which the food and the water were given to the animals *ad libitum.* The body temperature at 0, 1, 2, 4, 6, 8 hours after the administration was measured. The results are shown in Fig. 12 (in the figure, -Δ-: BDNF-treated group, and -■-: vehicle-treated group).

As is shown in Fig. 12, the body temperature of *db/db* mice being exposed to cold place was quickly dropped in the vehicle-treated group. On the other hand, the decreased body temperature found in the vehicle-treated group was significantly inhibited in the BDNF-treated group. It was confirmed that the inhibitory effect of BDNF against decreased body temperature of *db/db* mice was effective not only under fasting but also under cold conditions.

### Example 6:

*db/db* Mice were grouped into two groups so that the HbAlc values and the body weights were not biased in each group (5 animals/group). Group 1: vehicle-treated group, and Group 2: BDNF-treated group. To the mice to which the food and water had not given overnight, BDNF was subcutaneously administered once at a dose of 20 mg/kg, and 4 hours thereafter, a solution of D-[U-¹⁴C]glucose (5 µCi, NEN Co., Ltd.) in physiological saline (200 µl) was intravenously injected under ether anesthesia. Immediately after the administration, each mouse as kept individually to a sealed metabolic cage (Metabolica, Sugiyamagen Iriki). Air was circulated in the metabolic cage at a constant flow rate (about 200 ml/min), and a trap tube containing 10 % NaOH solution was connected to the exit from the cage. The radioactivity of ¹⁴C of CO₂ released in exhalation was measured with time at 0, 15, 30, 60, 90, and 120 minutes after the D-[U-¹⁴C]glucose administration. The collected 10 % NaOH solution (1 ml) was mixed with a liquid scintillation (5 ml; Hionicfluor, Hewlett-Packard Co.), and the radioactivity of ¹⁴C of the mixture was measured with using a liquid scintillation counter (Hewlett-Packard Co.). The results are shown in Fig. 13 (in the figure, -Δ-: BDNF-treated group; -■-: vehicle-treated group).

As is shown in Fig. 13, there was a significant increase in carbohydrate metabolism (oxidization of glucose) by BDNF administration during the period of from 30 minutes after D-[U-¹⁴C]glucose administration to 120 minutes after D-[U-¹⁴C]glucose administration, at which the analysis was complete.

### INDUSTRIAL APPLICABILITY

The present agent for treatment of autonomic neuropathy can activate the autonomic nervous system, especially the sympathetic nervous system, which is lowered by various causes, and can exhibit an activity of improving decreased energy metabolism of the peripheral organs. By administering the present agent, it may be possible to treat patients with autonomic neuropathy such as orthostatic hypotension or thermoregulation disorders, which have been difficult to be treated.

The agent for improving energy metabolism of the present invention can also be used as an agent for treatment of lipid metabolic disorder or derangement of carbohydrate metabolism. Further, the agent for improving thermogenesis of the present invention can also be used as an agent for improving cold constitution or hypothermia, an agent for prevention of decreased body temperature in cold place, or as an agent for normalizing decreased body temperature, or as an agent for prevention of frostbite.

## Claims

1. An agent for treatment of autonomic neuropathy, which comprises as the active ingredient a neurotrophic factor.

2. An agent for treatment of autonomic imbalance, which comprises as the active ingredient a neurotrophic factor.

3. The agent for treatment of autonomic imbalance according to claim 2, wherein the autonomic imbalance is induced by autonomic neuropathy.

4. The agent for treatment of autonomic neuropathy or autonomic imbalance according to claim 1 or claim 3, wherein the autonomic neuropathy is a circulatory disease, respiratory disorder, gastrointestinal disorder, urinary disorder, thermoregulation disorder, pupillary disorder, or neuroendocrine disorder.

5. An agent for treatment of autonomic neuropathy or autonomic imbalance induced by stress, which comprises as the active ingredient a neurotrophic factor.

6. The agent for treatment of autonomic neuropathy according to any one of claims 1 to 5, wherein the neurotrophic factor is a trkA, a trkB and/or a trkC receptor agonist.

7. The agent for treatment of autonomic neuropathy according' to claim 1, wherein the autonomic neuropathy is diabetic autonomic neuropathy, thermoregulation disorder, circulatory disease, or orthostatic hypotension.

8. An agent for inhibiting norepinephrine content decrease in peripheral tissue, which comprises as the active ingredient a neurotrophic factor.

9. The agent for inhibiting norepinephrine content decrease in peripheral tissue according to claim 11, wherein the peripheral tissue is brown adipose tissue, heart and/or muscles.

10. An agent for improving energy metabolism, which comprises as the active ingredient a neurotrophic factor.

11. An agent for improving thermogenesis, which comprises as the active ingredient a neurotrophic factor.

12. Use of a neurotrophic factor in preparation of an agent for treatment of autonomic neuropathy or autonomic imbalance.

13. A method for treatment of autonomic neuropathy or autonomic imbalance, which comprises administering a therapeutically effective amount of a neurotrophic factor to a patient with autonomic neuropathy or autonomic imbalance.
